# EUROPÄISCHE PATENTANMELDUNG

(11) **EP 4 740 889 A1**
(43) Veröffentlichungstag der Anmeldung: **13.05.2026**
(21) Anmeldenummer: 25212237.9
(22) Anmeldetag: 30.10.2025
(51) Int. Cl.: A61B 34/30, A61B 34/35, A61B 34/37, A61B 34/00, A61B 90/50, B25J 9/00

(54) **ROBOTERBASIS UND MEDIZINISCHES OPERATIONSSYSTEM**

(30) Priorität: 11.11.2024 DE 102024132852
(71) Anmelder: Karl Storz SE & Co. KG, 78532 Tuttlingen (DE)
(72) Erfinder: Hoffmann, Martin, 78532 Tuttlingen (DE)

(57) **Zusammenfassung**

Die vorliegende Erfindung betrifft eine Roboterbasis (10) umfassend eine erste Roboterarmschnittstelle (12), welche zur Ankopplung eines ersten Roboterarms (14) eingerichtet ist, und zumindest eine zweite Roboterarmschnittstelle (16), welche zur Ankopplung eines zweiten Roboterarms (18) eingerichtet ist, wobei die erste Roboterarmschnittstelle (12) und die zweite Roboterarmschnittstelle (16) jeweils zur Ankopplung eines Software-RCM-Roboterarms (20) eingerichtet sind. Die vorliegende Erfindung betrifft ferner eine Roboterbasis (10) mit einem Haltearm (22), an welchem die erste Roboterarmschnittstelle (12) und die zweite Roboterarmschnittstelle (16) in einer fixierten relativen Lage zueinander ausgebildet sind.

## Beschreibung

Die vorliegende Erfindung betrifft eine Roboterbasis und ein medizinisches Operationssystem.

Aus dem Stand der Technik sind Roboterbasen für medizinische Operationssysteme bekannt, welche mehrere Roboterarme tragen. An den Roboterarmen ist jeweils distal ein medizinisches Instrument wie ein Endoskop, ein chirurgisches Werkzeug oder dergleichen angebracht, mittels derer ein chirurgischer Eingriff minimalinvasiv durchgeführt werden kann.

Zur Durchführung des minimalinvasiven Eingriffs unter Verwendung eines solchen medizinischen Operationssystems wird der Patient auf einem Operationstisch fixiert. Das Operationssystem wird neben dem Operationstisch positioniert und die Roboterarme individuell für den Patienten ausgerichtet und eingestellt.

Derartige medizinische Operationssysteme aus dem Stand der Technik weisen üblicherweise einen sogenannten "Hardware Remote Center of Motion" (Hardware-RCM) auf. Ein Hardware-RCM beschreibt einen virtuellen Drehpunkt, um den sich ein Roboterarm oder ein am Roboterarm befestigtes medizinisches Instrument bewegt. Dieser Drehpunkt bleibt während der Bewegung stationär. Der Hardware-RCM wird mithilfe einer speziell konzipierten mechanischen Struktur erreicht und beruht beispielsweise auf der Kinematik eines Parallelogramms. Parallelkinematische Roboter sind etwa in der US 5 697 939 A, der US 5 817 084 A, der US 6 902 560 B1 und der US 2016/0100900 A1 beschrieben.

Ein am Roboter befestigtes und zur Durchführung des Eingriffs in den Körper des Patienten eingeführtes medizinisches Instrument wird um diesen Hardware-RCM bewegt und in einer Vorbereitungsprozedur derart eingestellt, dass er an einem Zugangspunkt des medizinischen Instruments in den Körper liegt. Dadurch kann eine präzise Kontrolle des an dem jeweiligen Roboterarm fixierten medizinischen Instruments gewährleistet werden, wobei Kräfte, die auf die Haut oder das Gewebe des Patienten ausgeübt werden, minimiert werden.

Der vorliegenden Erfindung liegt die Erkenntnis zugrunde, dass diese herkömmlichen medizinischen Operationssysteme aufgrund der notwendigen Mechanik zur Bereitstellung des Hardware-RCM sperrig sind und ein hohes Gewicht aufweisen. Dies macht den Einsatz der bekannten medizinischen Operationssystemen in verhältnismäßig kleinen Operationssälen zum Teil unmöglich und setzt zudem eine hohe Mindesttragkraft eines Bodens, auf dem das System aufgestellt werden soll, voraus. Darüber hinaus erschwert die Größe des Systems auch bei größeren Operationssälen den Zugang eines Arztes zum Patienten. Dies ist gerade für die Anästhesie ungünstig. Ferner wurde durch die Erfinder erkannt, dass bei der Konversion zu einem offenen Eingriff, die beispielsweise aufgrund einer Komplikation notwendig sein kann, der Zeitbedarf, um ein herkömmliches medizinisches Operationssystem vom Patienten zu entfernen, aufgrund der Größe des Systems zu groß ist.

Ausgehend vom Stand der Technik liegt der Erfindung die Aufgabe zugrunde, ein kompaktes medizinisches Operationssystem bereitzustellen.

Die Aufgabe wird erfindungsgemäß durch eine Roboterbasis und ein medizinisches Operationssystem gelöst, wie sie hierin beschrieben und in den Ansprüchen definiert sind.

Die vorliegende Erfindung sieht vor, eine Roboterbasis, insbesondere für ein medizinisches Operationssystem, bereitzustellen. Diese umfasst eine erste Roboterarmschnittstelle, welche zur Ankopplung eines ersten Roboterarms eingerichtet ist, und zumindest eine zweite Roboterarmschnittstelle, welche zur Ankopplung eines zweiten Roboterarms eingerichtet ist.

Gemäß einem Aspekt der Erfindung sind die erste Roboterarmschnittstelle und die zweite Roboterarmschnittstelle jeweils zur Ankopplung eines Software-RCM-Roboterarms eingerichtet.

Gemäß einem weiteren Aspekt der Erfindung umfasst die Roboterbasis, insbesondere zusätzlich zu den Merkmalen des voranstehend beschriebenen Aspekts, einen Haltearm, an welchem die erste Roboterarmschnittstelle und die zweite Roboterarmschnittstelle in einer fixierten relativen Lage zueinander ausgebildet sind.

Die vorliegende Erfindung sieht ferner vor, ein medizinisches Operationssystem bereitzustellen. Dieses umfasst eine erfindungsgemäße Roboterbasis und einen ersten Software-RCM-Roboterarm, welcher mit der ersten Roboterarmschnittstelle gekoppelt ist.

Durch diese Merkmale kann ein kompaktes medizinisches Operationssystem bereitgestellt werden. Die erfindungsgemäße Roboterbasis weist kleine Abmessungen auf und ist wenig raumgreifend, insbesondere im Vergleich zu herkömmliche Roboterbasen für Hardware-RCM-Roboterarme. Dadurch kann eine Gewichtsersparnis erzielt werden, sowohl absolut als auch relativ zu einer Basisfläche einer Aufstelleinheit, mittels derer die Roboterbasis auf einem Boden eines Operationssaals aufgestellt ist. Die erfindungsgemäße Roboterbasis und das entsprechende medizinische Operationssystem, das diese Roboterbasis verwendet, können also in einem größeren Spektrum an Operationssälen verwendet werden. In anderen Worten wird zur Verwendung der Roboterbasis und des Operationssystems eine kleinere Anforderung hinsichtlich räumlicher Größe und Bodentragkraft an den Operationsaal gestellt im Vergleich zu den herkömmlichen Operationssystemen. Überdies hat die Verwendung von Software-RCM-Roboterarmen den Vorteil, dass das Operationssystem eine große Flexibilität hinsichtlich der Bewegung der Roboterarme und der Abstimmung auf den Patienten aufweist. Dies hat gemeinsam mit der großen Kompaktheit der Roboterbasis und des Operationssystems zur Folge, dass ein Arzt im Notfall schneller zu dem Patienten gelangen kann, beispielsweise um lebensrettende Maßnahmen einzuleiten. Ferner ist die Roboterbasis flexibler im Operationsaal aufstellbar und ein Aufbau des Operationssystems kann schnell durchgeführt werden.

Das medizinische Operationssystem kann ein, insbesondere robotergestütztes, medizinisches Operationssystem beispielsweise zur Durchführung minimalinvasiver Eingriffe bzw. für minimalinvasive Chirurgie sein. Ein solcher Eingriff ist insbesondere ein operatives, chirurgisches Verfahren, das innerhalb einer Körperhöhle eines Patienten durchgeführt wird. Ein Beispiel für einen solchen Eingriff ist der Magenbypass (engl. *gastric bypass*)*.* Bei diesen Eingriffen werden, wie bereits beschrieben, mehrere medizinische Instrumente über eine lediglich kleine Inzision der Körperhöhle zugeführt. Die medizinischen Instrumente sind an Roboterarmen angeordnet und/oder an diesen ausgebildet. Über eine Steuerung der Roboterarme können entsprechend die medizinischen Instrumente innerhalb der Körperhöhle positioniert und gesteuert werden.

Eine Roboterbasis kann eine zentrale Plattform eines medizinischen, insbesondere robotergestützten, Operationssystems sein. Sie sorgt beispielsweise für die notwendige Stabilität zur Stützung der Roboterarme, kann zur Schwingungsisolierung bzw. zur Entkopplung vom Boden ausgebildet sein und kann eine verkippsichere Aufstellung und Einstellung der Roboterarme ermöglichen. Dadurch werden präzise, akkurate Bewegungen der Roboterarme während chirurgischer Eingriffe ermöglicht. In anderen Worten kann in der Roboterbasis eine mechanische Aufstelleinheit gesehen werden.

Wenn die Roboterarme des Operationssystems beispielsweise nicht über der Basisfläche der Roboterbasis positioniert sind, kann die Roboterbasis ein Gegengewicht zu den Roboterarmen bilden. Dies ist insbesondere hinsichtlich des Gewichts der Roboterarme von Bedeutung. Sind die Roboterarme mit einem geringen Gewicht ausgebildet, kann eine kleine, leichte Roboterbasis vorgesehen sein. Vorliegend werden Software-RCM-Roboterarme verwendet, welche kompakt und leicht ausgebildet werden können. Dadurch wird erreicht, dass die Roboterbasis zum einen ein relativ geringes Gegengewicht zu den Roboterarmen bereitstellen muss und zum anderen eine verhältnismäßig kleine Basisfläche benötigt.

Gemäß einigen Ausführungsformen kann die Robotereinheit und/oder das medizinische Operationssystem in einem Operationssaal oder ähnlichen Räumlichkeiten bewegbar sein bzw. in einer Operationsumgebung bewegbar und relativ zu einem Patienten positionierbar sein. Üblicherweise wird ein Patient auf einem Operationstisch fixiert bzw. befindet sich der Patient während eines Eingriffs auf dem Operationstisch. Die Roboterbasis kann, insbesondere gemeinsam mit an ihr angebrachten Roboterarmen, relativ zu diesem Operationstisch positioniert werden, beispielsweise nachdem der Patient für den Eingriff vorbereitet wurde. In diesem Zusammenhang wird durch das kompakte Operationssystem und die kompakte Roboterbasis eine große Flexibilität und damit einhergehend eine Zeitersparnis beim Vorbereiten des Eingriffs erzielt. Ferner ist der Zugang zum Patienten während des Eingriffs durch die Kompaktheit offen.

In der Roboterbasis können weitere Funktionseinheiten untergebracht sein, beispielsweise eine Steuereinheit, eine oder mehrere Versorgungseinheiten, Energiespeicher und/oder dergleichen. Mittels der Steuereinheit und/oder der zumindest einen Versorgungseinheit können die Roboterarme bzw. an den Roboterarmen fixierte medizinische Instrumente betreibbar sein.

Unter einem Roboterarm kann eine bewegliche Vorrichtung verstanden werden, die durch mehrere Achsen steuerbar ist und präzise Bewegungen in verschiedene Richtungen ausführen kann. Er kann dafür ausgebildet sein, eines oder mehrere medizinische Instrumente wie ein chirurgisches Werkzeug, etwa eine Zange, ein HF-Werkzeug, einen Laserkopf und/oder dergleichen sowie ein Endoskop zu tragen und zu positionieren. Diese Instrumente sind in eine Körperhöhle des Patienten zur Durchführung eines minimalinvasiven Eingriffs einführbar. Üblicherweise werden für einen minimalinvasiven Eingriff mehrere medizinische Instrumente verwendet. Diese sind häufig an unterschiedlichen Roboterarmen vorgesehen. Die Roboterarme können während des Eingriffs koordiniert und aufeinander abgestimmt bewegbar und gemeinsam ansteuerbar sein. Jedes Instrument kann etwa über einen individuellen oder auch über einen gemeinsamen Zugangspunkt, welcher beispielsweise eine Inzision sein kann, in die Körperhöhle eingebracht werden.

Wird beispielsweise ein Eingriff im Bauchraum durchgeführt, kann durch einen Arzt gezielt eine kleine Inzision in die Bauchdecke eingebracht werden, durch die ein Instrument der Bauchhöhle zugeführt werden kann. Üblicherweise wird das Instrument um diesen Zugangspunkt in der Bauchdecke rotiert, um Belastungen und Verletzungen des Gewebes der Bauchdecke zu verhindern. Unter diesem Zugangspunkt kann ein sogenannter Trokarpunkt verstanden werden. Das medizinische Instrument kann folglich im Bauchraum in einem kegelförmigen Bereich bewegt werden, wobei der Trokarpunkt die Spitze des Kegels beschreibt.

Ein Roboterarm kann entsprechend dazu eingerichtet sein, um den Trokarpunkt bewegt zu werden. Dieser kann sich von dem Roboterarm beabstandet im Raum befinden. Der Trokarpunkt kann für jeden Roboterarm individuell eingestellt werden, etwa bei der Vorbereitung der Operation. Wie bereits beschrieben, ist es wichtig, den Roboterarm um diesen Trokarpunkt zu rotieren und Linearbewegungen durch den Trokarpunkt durchzuführen. Andernfalls besteht die Gefahr einer Verletzung des Patienten.

Wie bereits beschrieben ist ein Hardware-RCM-Roboterarm um den RCM bewegbar. Der RCM kann entsprechend den obigen Ausführungen den Trokarpunkt abbilden und der Roboterarm bzw. ein am Roboterarm angeordnetes medizinisches Instrument um den RCM rotiert werden.

Ein Software-RCM-Roboterarm kann ebenso um den Trokarpunkt bewegbar sein. Im Unterschied zum Hardware-RCM-Roboterarm kann dies nicht durch die spezielle mechanische Konstruktion realisiert werden, sondern etwa durch eine softwarebasierte Steuerung des Roboterarms. Das bedeutet, dass der feste Drehpunkt des medizinischen Instruments, durch den sich Bewegungen präzise um einen bestimmten Punkt (z. B. den Trokarpunkt) vollziehen sollen, beispielsweise durch Regelalgorithmen und Software-Steuerung bestimmt und eingehalten wird.

Statt mechanische Vorrichtungen wie eine Parallelkinematik zu verwenden, kann die stattdessen verwendete Software die erforderlichen Bewegungen der Roboterarme berechnen und diese kontinuierlich anpassen, um sicherzustellen, dass der gewählte RCM bzw. Trokarpunkt stabil bleibt und eingehalten wird.

Eine Roboterarmschnittstelle kann zur mechanischen Fixierung bzw. zum Befestigen des Roboterarms an der Roboterbasis vorgesehen sein. Zusätzlich kann über die Roboterarmschnittstelle der Roboterarm elektrisch an die Roboterbasis anbindbar sein. Über die Roboterarmschnittstelle kann also beispielsweise elektrische Energie übertragbar sein und/oder können Steuersignale übertragbar sein. Entsprechend kann der Roboterarm elektrisch über die Roboterarmschnittstelle mit einer Roboterarmsteuereinheit verbindbar sein.

Die Roboterarmschnittstelle kann beispielsweise gemeinsam mit einem Halteabschnitt des Roboterarms eine Verbindungseinheit ausbilden. Der Halteabschnitt kann einen proximalen Abschnitt des Roboterarms definieren. An einem distalen Abschnitt kann ein medizinisches Instrument angeordnet sein.

Beispielsweise kann die Verbindungseinheit als eine Art Bajonettverschluss ausgebildet sein. Alternativ oder zusätzlich kann die Verbindungseinheit einen Klemmmechanismus aufweisen und/oder einen Schraubmechanismus. Andere Fixierungsarten des Roboterarms an der Roboterbasis über die Roboterarmschnittstelle sind ebenso denkbar.

Unter dem Haltearm kann eine Trägereinheit der Roboterbasis für mehrere Roboterarme verstanden werden. Der Haltearm kann entsprechend strukturell derart ausgebildet sein, dass die Roboterarme sicher bzw. starr zueinander, insbesondere hinsichtlich der Halteabschnitte der Roboterarme, gehalten werden können. Insbesondere wird unter dem Haltearm eine balkenförmige Struktur verstanden bzw. kann der Haltearm balkenförmig ausgebildet sein. Der Haltearm kann also eine längliche Struktur sein, die etwa entlang einer Achse eine größere Erstreckung aufweist als entlang der senkrecht zu dieser Achse stehenden Achsen. Diese Achse kann entsprechend eine Haupterstreckungsachse sein. Entlang dieser Achse kann die Erstreckung des Haltearms beispielsweise zumindest zweimal, insbesondere zumindest dreimal, bevorzugt zumindest viermal so groß sein, wie entlang einer senkrecht zu dieser Haupterstreckungsachse liegenden Achse.

Der Haltearm kann derart ausrichtbar sein, dass er sich von der Basisfläche der Roboterbasis wegstreckt. Mittels des Haltearms können die Roboterarmschnittstellen in einem Raum über einer Fläche positioniert werden, die von der Basisfläche beabstandet ist. Dazu kann der Haltearm insbesondere an einem Ende mit anderen Bestandteilen der Roboterbasis verbunden sein. Das entgegengesetzte Ende des Haltearms kann frei im Raum bewegbar sein. Mit dem "Ende" und dem "entgegengesetzten Ende" sind insbesondere die Enden entlang der Längsrichtung des Haltearms gemeint.

Eine Lage der ersten Roboterarmschnittstelle relativ zur zweiten Roboterarmschnittstelle kann fixiert sein. Vorteilhafterweise können also beide Roboterarmschnittstellen gemeinsam im Raum ausgerichtet werden. Eine Einstellbarkeit des medizinischen Operationssystems wird dadurch vereinfacht. Mit der Lage kann eine Position im Raum und/oder eine Winkelorientierung gemeint sein. In anderen können die Roboterarme unbeweglich zueinander vorgesehen sein und/oder jeweils unbeweglich an einer vorgesehenen Position an der Roboterbasis angeordnet sein. Ist der Haltearm vorgesehen, sind entsprechend dem weiter oben angeführten Merkmal die erste Roboterarmschnittstelle und die zweite Roboterarmschnittstelle in einer fixierten relativen Lage zueinander an dem Haltearm ausgebildet. Durch eine Positionierung des Haltearms relativ zu anderen Komponenten der Roboterbasis, insbesondere einem Hauptkörper der Roboterbasis, können die Roboterarmschnittstellen also im Raum positioniert werden.

Eine kompakte und flexibel einsetzbare Roboterbasis kann bereitgestellt werden, wenn die erste Roboterarmschnittstelle und die zweite Roboterarmschnittstelle entlang einer Längsachse des Haltearms nebeneinander angeordnet sind. In anderen Worten können die Roboterarmschnittstellen entlang der Längserstreckungsrichtung bzw. entlang der Haupterstreckungsachse des Haltearms vorgesehen sein.

Die Roboterbasis kann ferner eine dritte Roboterarmschnittstelle, welche zur Ankopplung eines dritten Roboterarms eingerichtet ist, umfassen. Die dritte Roboterarmschnittstelle kann, insbesondere gemäß obigen Ausführungen, in einer fixierten relativen Lage zu der ersten und zweiten Roboterarmschnittstelle an dem Haltearm ausgebildet sein. Es kann also ein weiterer Roboterarm an dem Haltearm vorgesehen sein. Eine Anzahl an medizinischen Instrumenten kann dadurch erhöht werden, wodurch komplexere Eingriffe ermöglicht werden. Die Roboterbasis kann gemäß einigen Ausführungsformen genau drei Roboterarmschnittstellen aufweisen. Diese können an dem Haltearm ausgebildet sein und entsprechend gemeinsam durch Positionierung des Haltearms im Raum bewegbar sein.

Gemäß einer Weiterbildung sind die erste Roboterarmschnittstelle, die zweite Roboterarmschnittstelle und die dritte Roboterarmschnittstelle äquidistant zueinander entlang der Längsachse nebeneinander an dem Haltearm angeordnet. Dadurch kann eine gleichmäßige Verteilung der auf den Haltearm wirkenden Kräfte und eine optimierte Raumnutzung erreicht werden. Dadurch kann der Haltearm gewichtsoptimiert ausgebildet sein, wodurch eine kompaktere Roboterbasis bereitstellbar ist. Die äquidistante Anordnung führt ferner zu einer verbesserten Stabilität des Systems und erleichtert die Kollisionsvermeidung zwischen den Roboterarmen, wodurch präzise und ungehinderte Bewegungen während eines chirurgischen Eingriffs ermöglicht werden.

Ein kompaktes Operationssystem und eine effizient im Raum bewegbare Roboterbasis können bereitgestellt werden, wenn der Haltearm schwebend über einem Operationstisch anordenbar ist, insbesondere derart, dass die Roboterarmschnittstellen in einem gedachten sich senkrecht oberhalb des Operationstischs erstreckenden und seitlich durch den Operationstisch begrenzten Raum anordenbar sind. In einer Verstaukonfiguration kann das medizinische Operationssystem etwa raumoptimiert angeordnet sein. Beispielsweise können die Roboterarme eng an der Roboterbasis und/oder der Haltearm raumsparend gelagert sein. In einer Anwendungskonfiguration kann sich der Haltearm von dem Hauptkörper der Roboterbasis wegerstrecken. Bedarfsweise kann der Haltearm also über den Operationstisch bewegbar sein, um das Operationssystem in die Anwendungskonfiguration zu überführen. Unter "schwebend" kann verstanden werden, dass der Haltearm sich von dem Hauptkörper der Roboterbasis wegerstreckend angeordnet ist. In anderen Worten ist der Haltearm in einem Zustand angeordnet, in dem er sich seitlich von dem Hauptkörper wegstreckt. Ist der Hauptkörper mit seiner Basisfläche neben dem Operationstisch angeordnet, kann sich der Haltearm entsprechend etwa quer über den Operationstisch und über einem auf dem Operationstisch liegenden Patienten erstrecken, dies insbesondere in der Anwendungskonfiguration.

Ferner kann die Roboterbasis eine Aufstelleinheit zur Aufstellung auf einem Boden umfassen. Der Haltearm kann mit der Aufstelleinheit verbunden sein und bezüglich der Aufstelleinheit zumindest drei und insbesondere genau drei Freiheitsgrade aufweisen. Durch die Positionierbarkeit des Haltearms mit drei Freiheitsgraden können die Roboterarme flexibel bzgl. des Patienten und dem Operationstisch ausgerichtet werden. Durch die Bewegungsmöglichkeit des Haltearms kann erreicht werden, dass eine kompakte Roboterbasis verwendet werden kann, die dennoch flexibel anwendbar ist und ein breites Spektrum an Eingriffen ermöglicht. Der Hauptkörper der Roboterbasis kann die Aufstelleinheit umfassen und/oder definieren. Die Aufstelleinheit kann ferner die Basisfläche aufweisen und/oder definieren.

Eine Höheneinstellbarkeit des Haltearms kann erreicht werden, wenn der Haltearm linear in vertikaler Richtung relativ zur Aufstelleinheit verfahrbar ist, insbesondere entlang einer vertikalen Achse, welche sich durch die Aufstelleinheit erstreckt. Die Roboterarmschnittstellen sind dadurch relativ zum Patienten in einer Höhenlage verstellbar. Dadurch eröffnet sich zum Beispiel die Möglichkeit, das Operationssystem flexibel örtlichen Begebenheiten anzupassen sowie die Roboterarme hinsichtlich einer Liegeposition des Patienten auszurichten. Der Patient kann also waagerecht, aber auch schräg liegen, wobei durch die Höhenverstellbarkeit auch in der Schräglage des Patienten ein Eingriff im Kopfbereich sowie im Beinbereich durchführbar ist. Der Haltearm kann in eine Höhenposition in einem Bereich von beispielsweise 500 nm bis 2500 nm, insbesondere 700 mm bis 2000 mm, bevorzugt 900 nm bis 1500 mm, verfahrbar sein. Mit der Höhenposition kann insbesondere ein Abstand eines Kopplungspunkts des Haltearms mit anderen Komponenten der Roboterbasis von dem Boden, auf dem die Roboterbasis aufgestellt ist, gemeint sein. Dadurch, dass sich die vertikale Achse durch die Aufstelleinheit erstreckt, kann eine gute Statik und Stabilität des Operationssystems erreicht werden. Beispielsweise kann die Roboterbasis eine ausfahrbare Säule umfassen, an der der Haltearm beweglich befestigt ist. Die Säule kann teleskopartig aus der Aufstelleinheit senkrecht herausfahrbar sein. Die Säule kann an ihrem distalen Abschnitt eine Konstruktion aufweisen, mittels derer der Haltearm gehalten und bewegbar gelagert ist.

Gemäß einigen Ausführungsformen ist der Haltearm derart an der Aufstelleinheit angeordnet, dass sich die Längsrichtung des Haltearms orthogonal zu der vertikalen Achse erstreckt. In anderen Worten ist erstreckt sich die Hauptachse des Haltearms senkrecht zur vertikalen Richtung bzw. ist der Haltearm waagerecht angeordnet. Die Roboterarmschnittstellen können demnach in der Horizontalen nebeneinander, insbesondere äquidistant zueinander, angeordnet sein.

Gemäß einer Weiterbildung ist der Haltearm dadurch in einer horizontalen Ebene positionierbar, dass der Haltearm um eine vertikale Achse relativ zur Aufstelleinheit drehbar ist, welche sich insbesondere durch die Aufstelleinheit erstreckt. In anderen Worten kann der Haltearm um die vertikale Achse geschwenkt werden. Der Haltearm kann um 360 Grad um die vertikale Achse drehbar sein. Das heißt, der Haltearm kann eine beliebige Winkelposition in der horizontalen Ebene einnehmen. Dies insbesondere dann, wenn sich der Haltearm in der horizontalen Ebene erstreckt, insbesondere mit der Hauptachse bzw., wenn sich die Längsachse in der horizontalen Ebene erstreckt.

Ferner kann der Haltearm um seine Längsachse drehbar sein, wobei die Längsachse insbesondere senkrecht zu einer vertikalen Achse angeordnet ist, welche sich durch die Aufstelleinheit erstreckt. Die Roboterbasis kann dadurch flexibel einsetzbar sein. Beispielsweise könnte die Roboterbasis entweder links oder rechts einem Operationstisch aufgestellt werden. Entsprechend kann der Haltearm, dessen Längsachse sich horizontal erstreckt, über den Operationstisch geschwenkt werden. Eine Höhenposition kann entsprechend angepasst werden. Zudem kann der Haltearm um seine Längsachse gedreht werden, um die Roboterarmschnittstellen in die gewünschte Lage bezüglich des Operationstisches zu bringen. Entsprechend kann der Haltearm für die linke als auch rechte Position neben dem Operationstisch in eine gleichartige Ausrichtung bezüglich des Operationstisches gebracht werden. Die Roboterarmschnittstellen können also beispielsweise für beide Orientierungen zum selben Ende des Operationstischs zeigen.

Eine derartige Roboterbasis zeichnet sich also durch eine große Flexibilität auf. Es bestehen zum Einsatz geringe Anforderungen an die Operationsumgebung. Insbesondere kann der Platzbedarf reduziert werden.

Der Haltearm kann in den genannten drei Freiheitsgraden motorisiert bewegbar sein. Es können also Motoren vorgesehen sein, die gezielt zur Ausrichtung des Haltearms ansteuerbar sind. Ferner kann der Haltearm in einer bestimmen Ausrichtung haltbar sein. Beispielsweise kann die Roboterbasis, insbesondere je Freiheitsgrad, einen Arretierungsmechanismus umfassen, mittels dessen/derer der Haltearm fixiert werden kann.

Komplexe Eingriffe können ermöglicht und eine große Flexibilität des Operationssystems kann erreicht werden, wenn der Software-RCM-Roboterarm zumindest sieben Freiheitsgrade aufweist. Der Roboterarm kann dadurch unter einer großen Bewegungsfreiheit um die Trokarpunkt bzw. den RCM bewegt werden. Durch eine koordinierte Ansteuerung einzelner Gelenke bzw. Segmente des Roboterarms können mehrere Bewegungsmuster abgefahren werden, die allesamt das Einhalten des RCM erlauben. Ein Platzbedarf kann dadurch reduziert werden, da flexibel und schnell auf äußere Einflüsse reagiert werden kann. Beispielsweise kann der Roboterarm nahe einer Wand betrieben werden. Ebenso kann eine Kollision mit einem Arzt verhindert werden, indem der Roboterarm dem Arzt ausweicht, wobei trotzdem der RCM eingehalten wird. In anderen Worten kann der Roboterarm durch die zumindest sieben Freiheitsgrade einen großen Nullspace aufweisen. In einigen Ausführungsformen weist der Software-RCM-Roboterarm genau sieben Freiheitsgrade auf.

Der Nullspace eines Roboterarms kann sich auf die Menge der Bewegungen beziehen, die der Roboterarm durchführen kann, ohne eine Endeffektorposition oder -orientierung zu verändern. Das heißt, der RCM kann bei unterschiedlichen Bewegungen eingehalten werden. Ein entsprechender, insbesondere mehrgelenkiger, Roboterarm kann dazu mehr Freiheitsgrade als notwendig aufweisen, um eine bestimmte Position und Orientierung des Endeffektors zu erreichen. Der Nullspace enthält Bewegungen, die innerhalb dieser zusätzlichen Freiheitsgrade ausgeführt werden, während der Endeffektor stabil bleibt.

Beispielsweise kann bei einem Roboterarm mit sieben Freiheitsgraden eine spezifische Position des Endeffektors auf verschiedene Arten bzw. unter verschiedenen Bewegungen erreicht werden, indem die Gelenke unterschiedlich ausgerichtet werden. Diese unterschiedlichen Gelenkstellungen, die den Endeffektor unverändert lassen, gehören zum Nullspace. Solche Bewegungen im Nullspace können genutzt werden, um Kollisionen zu vermeiden, die Gelenkbelastung zu minimieren oder eine optimale Haltung des Roboterarms zu erreichen.

Sechs der Freiheitsgrade können Rotationsfreiheitsgrade sein. Dadurch kann ein großer Nullspace bei gleichzeitig kompakter Ausbildung des Roboterarms erreicht werden. Der Software-RCM-Roboterarm kann also sechs Gelenke aufweisen, mittels derer eine Winkelposition zweier benachbarter Segmente des Roboterarms eingestellt werden können. Drei der Rotationsfreiheitsgrade können eine Rotation benachbarter Segmente um eine Längsachse der Segmente ermöglichen. Wiederum drei der Rotationsfreiheitsgrade können ein Schwenken benachbarter Segmente senkrecht zu der Längsachse der Segmente ermöglichen. Der Roboterarm kann beispielsweise aus sieben Segmenten ausgebildet sein, wobei eines der Segmente den Halteabschnitt ausbildet. Ein weiterer Freiheitsgrad kann eine Linearbewegung ermöglichen. Die Linearbewegung kann beispielsweise mittels eines distalen Endsegments des Roboterarms ermöglicht werden. Dieses Segment kann eine Schiene aufweisen, entlang derer ein befestigtes medizinisches Instrument linear bewegbar ist. Das medizinische Instrument kann ferner einen Rotationsfreiheitsgrad um seine Längsachse aufweisen.

Das medizinisches Operationssystem kann einen zweiten Software-RCM-Roboterarm aufweisen, welcher entsprechend dem ersten Software-RCM-Roboterarm ausgebildet und mit der zweiten Roboterarmschnittstelle gekoppelt ist. Entsprechend kann das medizinische Operationssystem einen dritten Software-RCM-Roboterarm aufweisen. Dadurch, dass die Roboterarme einen großen Nullspace aufweisen, können die Roboterarme flexibel zueinander bewegt werden, wodurch eine Kollision der Roboterarme verhindert werden kann, ohne eine Anwendungsmöglichkeit der Roboterarme deutlich einzuschränken. Dadurch wird ein sicherer Betrieb des Operationssystem möglich, wobei eine breite Variation an Eingriffen durchführbar sind.

Im Folgenden wird die vorliegende Erfindung anhand der beigefügten Figuren beispielhaft beschrieben. Die Zeichnung, die Beschreibung und die Ansprüche enthalten zahlreiche Merkmale in Kombination. Der Fachmann wird die Merkmale zweckmäßigerweise auch einzeln betrachten und im Rahmen der Ansprüche sinnvoll in Kombination verwenden.

Falls von einem bestimmten Objekt mehr als ein Exemplar vorhanden ist, ist ggf. nur eines davon in den Figuren und in der Beschreibung mit einem Bezugszeichen versehen. Die Beschreibung dieses Exemplars kann entsprechend auf die anderen Exemplare von dem Objekt übertragen werden. Sind Objekte insbesondere mittels Zahlenwörtern, wie beispielsweise erstes, zweites, drittes Objekt etc. benannt, dienen diese der Benennung und/oder Zuordnung von Objekten. Demnach können beispielsweise ein erstes Objekt und ein drittes Objekt, jedoch kein zweites Objekt umfasst sein. Allerdings könnten anhand von Zahlenwörtern zusätzlich auch eine Anzahl und/oder eine Reihenfolge von Objekten ableitbar sein.

Es zeigen:
- Fig. 1: eine schematische perspektivische Darstellung eines medizinischen Operationssystems mit einer Roboterbasis und drei Software-RCM-Roboterarmen sowie einen Operationstisch;
- Fig. 2: eine schematische perspektivische Darstellung der Roboterbasis;
- Fig. 3: eine schematische Unteransicht des medizinischen Operationssystems mit der Roboterbasis sowie des Operationstisches;
- Fig. 4: eine schematische Darstellung eines der Software-RCM-Roboterarmen sowie eines distal an diesem angeordneten medizinischen Instruments;
- Fig. 5: eine schematische Darstellung des medizinischen Operationssystems und des Operationstisches in einer Vorderansicht; und
- Fig. 6: eine schematische Darstellung des medizinischen Operationssystems und des Operationstisches in einer Seitenansicht.

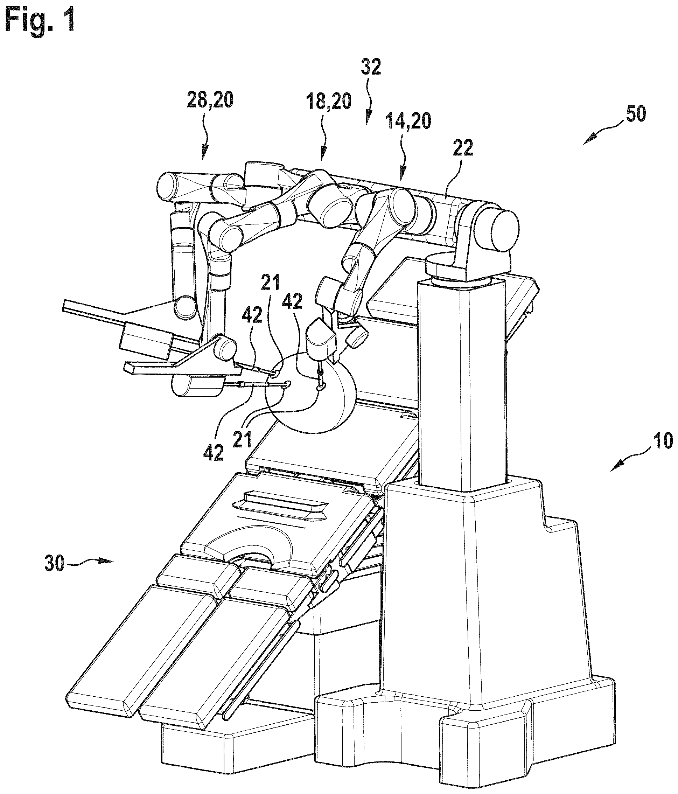

Die Fig. 1 zeigt eine schematische perspektivische Darstellung eines medizinischen Operationssystems 50 umfassend drei Software-RCM-Roboterarme 20 und eine Roboterbasis 10. Die drei Software-RCM-Roboterarme 20 sind an die Roboterbasis 10 angekoppelt, genauer an einen Haltearm 22 der Roboterbasis 10. Ferner ist ein Operationstisch 30 zu erkennen, auf dem ein Patient (nicht dargestellt) während eines an ihm durchgeführten Eingriffs liegt. Im dargestellten Fall ist der Operationstisch 30 schräg zur Horizontalen ausgerichtet.

Mittels des medizinischen Operationssystems 50 kann an dem Patienten ein minimalinvasiver Eingriff durchgeführt werden, in der dargestellten Konfiguration des medizinischen Operationssystems 50 etwa ein Magenbypass. Dazu ist an den Software-RCM-Roboterarmen 20 distal jeweils ein medizinisches Instrument 42 angeordnet. Dieses ist mittels des zugeordneten Software-RCM-Roboterarms 20 um einen entsprechenden Trokarpunkt 21 bewegbar. Der Trokarpunkt 21 stellt einen Zugang zu einer Körperhöhle bzw. der Bauchhöhle des Patienten dar, innerhalb derer der Eingriff durchgeführt werden soll. Während des Eingriffs soll keine Lateralbewegung des medizinischen Instruments 42 gegenüber dem zugeordneten Trokarpunkt 21 erfolgen, um eine Verletzung des Gewebes des Patienten zu vermeiden. Entsprechend kann ein RCM des zugeordneten Software-RCM-Roboterarms 20 softwarebasiert auf den Trokarpunkt gelegt werden, sodass dieser bei einer Bewegung das medizinische Instrument 42 um den Trokarpunkt 21 und entsprechend den RCM bewegt. Innerhalb der Körperhöhle kann eine Bewegung des medizinischen Instruments dadurch einen kegelförmigen Bereich abdecken, dessen Spitze am Trokarpunkt liegt.

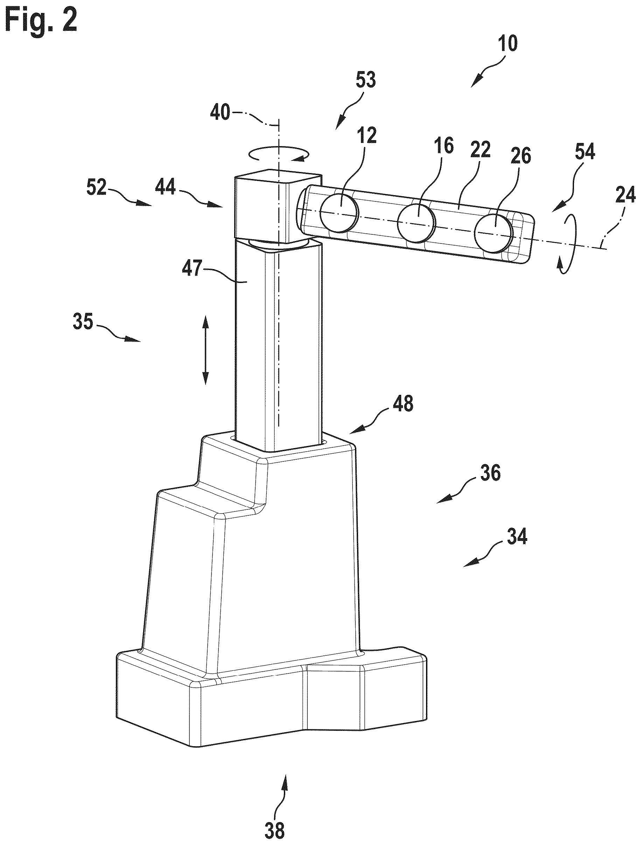

Der Haltearm 22 ist schwebend über dem Operationstisch 30 angeordnet. Das heißt, er erstreckt sich über dem Operationstisch 30 und quer zu diesem. Dadurch sind die Software-RCM-Roboterarm 20 oberhalb des Operationstisches 30 an den Haltearm 22 gekoppelt. Der Haltearm 22 umfasst drei Roboterarmschnittstellen (siehe z.B. Fig. 2), an die die Software-RCM-Roboterarme 20 gekoppelt sind. Diese Roboterarmschnittstellen sind also in einem gedachten sich senkrecht oberhalb des Operationstischs 30 erstreckenden und seitlich durch den Operationstisch 30 begrenzten Raum 32 angeordnet.

Die Software-RCM-Roboterarme 20 weisen jeweils sieben Freiheitsgrade auf, wie im Folgenden noch genauer beschrieben wird. Dadurch weisen sie einen großen Nullspace auf. Während des Eingriffs bieten sich dadurch mehrere unterschiedliche Bewegungen, wobei der Trokarpunkt 21 bzw. der RCM beibehalten wird. Dadurch kann eine Kollision der Software-RCM-Roboterarme 20 miteinander vermieden werden, wobei trotzdem eine hohe Flexibilität beibehalten wird.

Vor einem Eingriff kann der Patient auf dem Operationstisch 30 platziert und für den Eingriff vorbereitet werden. Erst nach erfolgter Vorbereitung muss das medizinische Operationssystem 50 neben dem Operationstisch 30 platziert werden. Da die Roboterbasis 10 zur Ankopplung der Software-RCM-Roboterarme 20 eingerichtet ist, sind das Operationssystem 50 und die Roboterbasis 10 kompakt ausgebildet. Dadurch ist das Platzieren des Operationssystem 50 einfach und effizient durchführbar. Es kann also eine Zeitersparnis der Vorbereitung des Eingriffs erzielt werden. Wie im Folgenden noch genauer beschrieben wird, weist der Haltearm 22 drei Freiheitsgrade auf, bzw. kann flexibel im Raum positioniert werden. Dadurch kann das Operationssystem 50 flexibel neben dem Operationstisch 30 platziert werden. Beispielsweise kann die Roboterbasis 10 links oder rechts vom Operationstisch 30 ohne Einschränkungen der Funktionalität platziert werden. Die Roboterarmschnittstellen können dann spezifisch für die relative Position der Roboterbasis 10 zu dem Operationstisch 30 durch Ausrichten des Haltearms 22 angeordnet werden.

Die Fig. 2 zeigt eine schematische perspektivische Darstellung der Roboterbasis 10. Die Roboterbasis 10 weist eine Aufstelleinheit 34 zur Aufstellung auf einem Boden auf. Die Aufstelleinheit 34 bildet einen Hauptkörper 36 der Roboterbasis 10. Ferner weist sie an ihrer Unterseite eine Basisfläche 38 auf, die in Kontakt mit dem Boden steht.

In dem Hauptkörper 36 ist eine Linearführung 48 ausgebildet, die sich entlang einer vertikalen Achse 40 erstreckt. Die vertikale Achse 40 verläuft durch die Roboterbasis 10. Die Linearführung 48 dient zur Linearführung einer verfahrbaren Säule 47 der Roboterbasis 10. Die Säule 47 kann motorisiert linear entlang der vertikalen Achse 40 verfahren werden. Dazu weist die Roboterbasis 10 einen nicht dargestellten Motor auf.

Die Roboterbasis 10 weist zudem den Haltearm 22 auf, an dem Roboterarmschnittstellen 12, 16, 26 angeordnet sind. An die Roboterarmschnittstellen 12, 16, 26 kann jeweils ein Software-RCM-Roboterarm 20 angekoppelt werden. An eine erste Roboterarmschnittstelle 12 kann ein erster Roboterarm 14, an eine zweite Roboterarmschnittstelle 16 ein zweiter Roboterarm 18 und an eine dritte Roboterarmschnittstelle 26 ein dritter Roboterarm 28 angekoppelt werden (siehe auch Fig. 1). Der Haltearm 22 ist balkenförmig ausgebildet und erstreckt sich entlang seiner Längsachse 24. Diese bildet eine Hauptachse des Haltearms 22. Die Roboterarmschnittstellen 12, 16, 26 sind nebeneinander entlang der Längsachse 24 in äquidistantem Abstand zueinander angeordnet. Die Mittelpunkte der Roboterarmschnittstellen 12, 16, 26 sind etwa 300 mm voneinander beabstandet und der balkenförmige Haltearm 22 etwa 700 mm lang. Die Lage der Roboterarmschnittstellen 12, 16, 26 zueinander ist also fixiert.

Die Säule 47 ist Teil einer Positioniereinheit 35, mittels derer der Haltearm 22 ausgerichtet werden kann. Die Positioniereinheit 35 bzw. die Roboterbasis 10 weist den Haltearm 22 auf. Dieser ist mit seinem proximalen Ende 53 mittels zweier Drehgelenke 44, 46 an einen distalen Abschnitt 52 der Säule 47 befestigt und in zwei Freiheitsgraden gegenüber der Säule 47 bewegbar. Mittels des ersten Drehgelenks 44 ist der Haltearm 22 um die vertikale Achse 40 rotierbar. Mittels des zweiten Drehgelenks 46 ist der Haltearm 22 um seine Längsachse 24 drehbar. Der Haltearm 22 wird also an seinem proximalen Ende 53 gehalten. Sein distales Ende 54 schwebt frei im Raum. Die Längsachse 24 des Haltearms 22 ist senkrecht zu der vertikalen Achse 40 angeordnet und erstreckt sich in einer horizontalen Ebene. Der Haltearm 22 erstreckt sich also seitlich von dem Hauptkörper 36 bzw. der Aufstelleinheit 34 weg. Ferner erkennt man, dass sich der Haltearm 22 größtenteils nicht über der Basisfläche 38 erstreckt. Der Haltearm 22 erstreckt sich von der Basisfläche 38 weg.

Die Drehgelenke 44, 46 sind ebenso motorisiert. Der Haltearm 22 kann also vollständig motorisiert im Raum ausgerichtet werden. Mittels der Säule 47 kann der Haltearm 22 also in seiner Höhe über dem Boden verstellt werden. Mittels des Drehgelenks 44 kann eine Winkelposition des Haltearms 22 in der horizontalen Ebene eingestellt werden und mittels des Drehgelenks 46 eine Orientierung der Roboterarmschnittstellen 12, 16, 26.

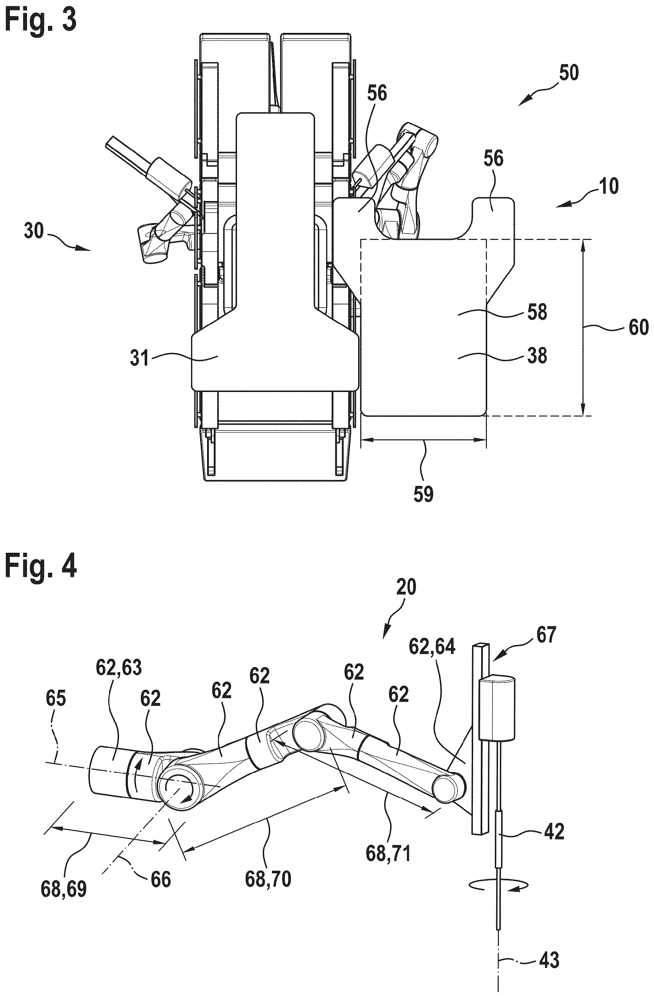

Die Fig. 3 zeigt eine schematische Unteransicht des medizinischen Operationssystems 50 mit der Roboterbasis 10 und des Operationstisches 30. Der Operationstisch 30 ist mit einer Aufstellfläche 31 auf einem Boden (nicht dargestellt) aufgestellt. Man erkennt, dass die Basisfläche 38 der Roboterbasis 10 in etwa so groß ist wie die Aufstellfläche 31. Diese Kompaktheit der Roboterbasis 10 kann dadurch erreicht werden, dass die Software-RCM-Roboterarme verwendet werden. Diese zeichnen sich durch eine große Kompaktheit und ein geringes Gewicht aus, insbesondere im Vergleich zu Hardware-RCM-Roboterarmen.

Die Basisfläche 38 weist eine rechteckige Grundfläche 58 mit einer Breite 59 von etwa 650 mm und einer Länge 60 von etwa 800 mm auf. An zwei Ecken einer Breitseite der Grundfläche 58 erstrecken sich seitlich sowie entlang der Längsrichtung der Grundfläche 58 zwei Vorsprünge 56 von der Grundfläche 58, die ebenfalls zur Basisfläche 38 gehören. Diese erstrecken sich in eine Vorzugsrichtung, in die die Roboterarmschnittstellen (siehe oben) des Haltearms bei einem Einsatz vorzugsweise ausgerichtet sind. Dadurch verhindern sie ein Verkippen der Roboterbasis 10 bei gleichzeitig platzsparender Ausgestaltung.

Die Fig. 4 zeigt eine schematische Ansicht eines der Software-RCM-Roboterarme 20. Die in der Fig. 1 gezeigten Roboterarme 20 sind identisch zueinander ausgebildet. Allerdings kann sich das an den jeweiligen Roboterarmen 20 befestigte medizinische Instrument 42 unterscheiden. Lediglich ein distaler Abschnitt des entsprechenden medizinischen Instruments 42 wird in die Körperhöhle eingebracht. Der Software-RCM-Roboterarme 20 wird so gesteuert, dass das medizinische Instrument 42 in bereits beschriebener Weise um den Trokarpunkt bewegt wird. Der Software-RCM-Roboterarm 20 weist sieben Freiheitsgrade auf. Sechs dieser Freiheitsgrade sind Rotationsfreiheitsgrade, einer ein Translationsfreiheitsgrad. Mittels des Translationsfreiheitsgrads ist das medizinische Instrument 42 durch den Trokarpunkt hindurch linear verschiebbar. Mittels der Rotationsfreiheitsgrade ist das medizinische Instrument 42 um den Trokarpunkt bewegbar, ohne dass eine seitliche Verschiebung stattfindet. Der Software-RCM-Roboterarm 20 ist aus mehreren Segmenten 62, genauer sieben Segmenten 62 ausgebildet, die zueinander beweglich gelagert sind. Insbesondere können die Segmente 62 zueinander motorisiert bewegt werden. Die dazu notwendigen Motoren können in dem Software-RCM-Roboterarm 20 aufgenommen sein.

Ein proximales Segment 62 ist als Halteabschnitt 63 ausgebildet. Mittels des Halteabschnitts 63 ist der Software-RCM-Roboterarm 20 an eine Roboterarmschnittstelle koppelbar. Die Kopplung ist starr ausgelegt. Das heißt, während eines Eingriffs findet ist keine Relativbewegung zwischen dem Halteabschnitt 63 und dem Haltearm vorgesehen.

Ein distales Segment 64 ist dazu vorgesehen, das medizinische Instrument 42 zu tragen. Dieses weist eine Linearführung 67 auf, wodurch der Translationsfreiheitsgrad bereitgestellt wird. Das medizinische Instrument 42 ist entlang der Linearführung 67 bewegbar.

Die sieben Segmente 62 sind drehbar miteinander gekoppelt. Zwischen jedem der Segmente 62 kann eine Rotationsbewegung durchgeführt werden. Die Art der Rotationsbewegung, bzw. die Achse, um die die Rotationsbewegung durchgeführt wird, ist alternierend von proximal nach distal vorgesehen. Das heißt, eine Rotationsbewegung ist um eine Längsachse 65 eines Segments 62 durchführbar. Die Rotationsbewegung, die durch die Kopplung mit einem darauffolgenden Segment ermöglicht wird, ist um eine senkrecht zu der Längsachse 65 liegenden Rotationsachse 66 durchführbar. Wiederum diesem Segment und dem darauffolgenden Segment ist wieder Rotationsbewegung um eine Segmentlängsachse durchführbar.

Zwei Segmente 62, das distale Segment 64 ausgenommen, bilden jeweils einen Roboterarmabschnitt 68. Der Halteabschnitt 63 und das direkt an diesen gekoppelte Segment 62 bilden einen ersten Roboterarmabschnitt 69. Dieser ist etwa 250 mm lang. Die darauffolgenden beiden Segmente 62 bilden einen zweiten Roboterarmabschnitt 70, welcher etwa 380 mm lang ist. Die wiederum darauffolgend beiden Segmente 62 bilden einen dritten Roboterarmabschnitt 71, welcher etwa 380 mm lang ist. Das distale Segment 64 ist etwa 470 mm lang. Es versteht sich, dass diese Maße je nach Roboterarm und Anwendungsfall unterschiedlich sein können und lediglich beispielhaft zu verstehen sind.

Das medizinische Instrument 42 kann wiederum einen Rotationsfreiheitsgrad aufweisen und eine Drehbewegung um seine Längsachse 43 ermöglichen. Ein am medizinischen Instrument 42 angebrachter Endeffektor (nicht im Detail dargestellt) kann dadurch innerhalb der Körperhöhle des Patienten ausgerichtet werden. Das medizinische Instrument 42 kann ein Endoskop umfassen. Eine Optik des Endoskops kann entsprechend ausgerichtet werden.

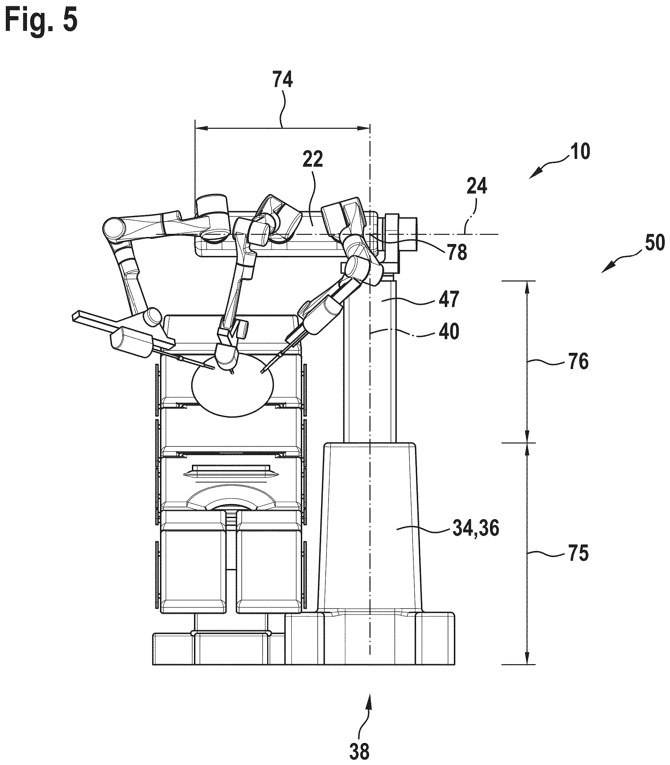

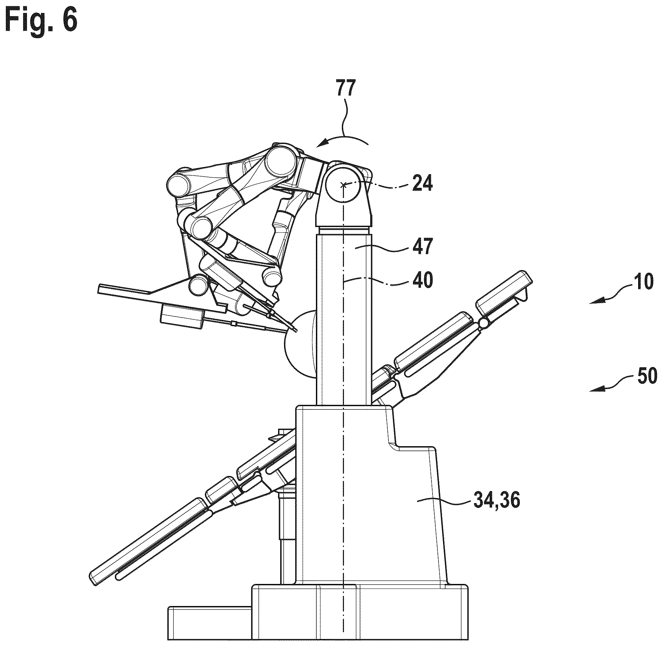

Die Figuren 5 und 6 zeigen weitere schematische Darstellungen des medizinischen Operationssystems 50 mit der Roboterbasis 10. Die Fig. 5 zeigt eine Vorderansicht, die Fig. 6 eine Seitenansicht. Man erkennt, dass sich der Haltearm 22 mit seiner Längsachse 24 senkrecht zu der vertikalen Achse 40 erstreckt. Der Haltearm 22 erstreckt sich seitlich über die Basisfläche 38 hinaus. Der gezeigte Haltearm 22 weist eine Länge 74 von etwa 700 mm auf. Ferner erkennt man den Hauptkörper 36 bzw. die Aufstelleinheit 34 der Roboterbasis 10. Diese weist eine Höhe 75 von etwa 800 mm auf. Die Säule 47 ist mit einer Länge 76 von etwa 500 mm aus dem Hauptkörper 36 bzw. der Aufstelleinheit 34 herausgefahren dargestellt. Der Schnittpunkt 78 der Längsachse 24 und der vertikalen Achse 40 kann durch das lineare Verfahren der Säule 47 innerhalb eines Abstandsbereichs über dem Boden von etwa 900 mm bis 1500 mm verfahren werden. In bereits beschriebener Weise kann der Haltearm 22 um die Längsachse 24 in Richtung des Pfeils 77 (siehe Fig. 6) rotiert werden.

### Bezugszeichenliste

- 10: Roboterbasis
- 12: erste Roboterarmschnittstelle
- 14: erster Roboterarm
- 16: zweite Roboterarmschnittstelle
- 18: zweiter Roboterarm
- 20: Software-RCM-Roboterarm
- 21: Trokarpunkt
- 22: Haltearm
- 24: Längsachse
- 26: dritte Roboterarmschnittstelle
- 28: dritter Roboterarm
- 30: Operationstisch
- 31: Aufstellfläche
- 32: gedachter Raum
- 34: Aufstelleinheit
- 35: Positioniereinheit
- 36: Hauptkörper
- 38: Basisfläche
- 40: vertikale Achse
- 42: medizinisches Instrument
- 44: erstes Drehgelenk
- 46: zweites Drehgelenk
- 47: Säule
- 48: Linearführung
- 50: medizinisches Operationssystem
- 52: distaler Abschnitt
- 53: proximales Ende
- 54: distales Ende
- 56: Vorsprung
- 58: Grundfläche
- 59: Breite
- 60: Länge
- 62: Segment
- 63: Halteabschnitt
- 64: distales Segment
- 65: Längsachse
- 66: Rotationsachse
- 67: Linearführung
- 68: Roboterarmabschnitt
- 69: erster Roboterarmabschnitt
- 70: zweiter Roboterarmabschnitt
- 71: dritter Roboterarmabschnitt
- 74: Länge
- 75: Höhe
- 76: Länge
- 77: Pfeil
- 78: Schnittpunkt

## Patentansprüche

1. Roboterbasis (10) umfassend:
- eine erste Roboterarmschnittstelle (12), welche zur Ankopplung eines ersten Roboterarms (14) eingerichtet ist, und
- zumindest eine zweite Roboterarmschnittstelle (16), welche zur Ankopplung eines zweiten Roboterarms (18) eingerichtet ist,
**dadurch gekennzeichnet, dass**
die erste Roboterarmschnittstelle (12) und die zweite Roboterarmschnittstelle (16) jeweils zur Ankopplung eines Software-RCM-Roboterarms (20) eingerichtet sind.

2. Roboterbasis (10) nach Anspruch 1, **dadurch gekennzeichnet, dass** eine Lage der ersten Roboterarmschnittstelle (12) relativ zur zweiten Roboterarmschnittstelle (16) fixiert ist.

3. Roboterbasis (10) nach dem Oberbegriff von und insbesondere nach einem der Ansprüche 1 oder 2, **gekennzeichnet durch** einen Haltearm (22), an welchem die erste Roboterarmschnittstelle (12) und die zweite Roboterarmschnittstelle (16) in einer fixierten relativen Lage zueinander ausgebildet sind.

4. Roboterbasis (10) nach Anspruch 3, **dadurch gekennzeichnet, dass** die erste Roboterarmschnittstelle (12) und die zweite Roboterarmschnittstelle (16) entlang einer Längsachse (24) des Haltearms (22) nebeneinander angeordnet sind.

5. Roboterbasis (10) nach Anspruch 3 oder 4, **gekennzeichnet durch:**
- zumindest eine dritte Roboterarmschnittstelle (26), welche zur Ankopplung eines dritten Roboterarms (28) eingerichtet ist,
wobei die dritte Roboterarmschnittstelle (26) in einer fixierten relativen Lage zu der ersten und zweiten Roboterarmschnittstelle (12, 16) an dem Haltearm (22) ausgebildet ist.

6. Roboterbasis (10) nach den Ansprüchen 4 und 5, **dadurch gekennzeichnet, dass** die erste Roboterarmschnittstelle (12), die zweite Roboterarmschnittstelle (16) und die dritte Roboterarmschnittstelle (26) äquidistant zueinander entlang der Längsachse (24) nebeneinander an dem Haltearm (22) angeordnet sind.

7. Roboterbasis (10) nach einem der Ansprüche 3 bis 6, **dadurch gekennzeichnet, dass** der Haltearm (22) schwebend über einem Operationstisch (30) anordenbar ist, insbesondere derart, dass die Roboterarmschnittstellen (12, 16, 26) in einem gedachten sich senkrecht oberhalb des Operationstischs (30) erstreckenden und seitlich durch den Operationstisch (30) begrenzten Raum (32) anordenbar sind.

8. Roboterbasis (10) nach einem der Ansprüche 3 bis 7, **gekennzeichnet durch:**
- eine Aufstelleinheit (34) zur Aufstellung auf einem Boden,
wobei der Haltearm (22) mit der Aufstelleinheit (34) verbunden ist und bezüglich der Aufstelleinheit (34) zumindest drei und insbesondere genau drei Freiheitsgrade aufweist.

9. Roboterbasis (10) nach Anspruch 8, **dadurch gekennzeichnet, dass** der Haltearm (22) linear in vertikaler Richtung relativ zur Aufstelleinheit (34) verfahrbar ist, insbesondere entlang einer vertikalen Achse (40), welche sich durch die Aufstelleinheit (34) erstreckt.

10. Roboterbasis (10) nach Anspruch 8 oder 9, **dadurch gekennzeichnet, dass** der Haltearm (22) um eine vertikale Achse (40) relativ zur Aufstelleinheit (34) drehbar ist, welche sich insbesondere durch die Aufstelleinheit (34) erstreckt.

11. Roboterbasis (10) nach einem der Ansprüche 3 bis 10, **dadurch gekennzeichnet, dass** der Haltearm (22) um seine Längsachse (24) drehbar ist, wobei die Längsachse (24) insbesondere senkrecht zu einer vertikalen Achse (40) angeordnet ist, welche sich durch die Aufstelleinheit (24) erstreckt.

12. Medizinisches Operationssystem (50) umfassend:
- eine Roboterbasis (10) nach einem der vorhergehenden Ansprüche und
- einen ersten Software-RCM-Roboterarm (14, 20), welcher mit der ersten Roboterarmschnittstelle (12) gekoppelt ist.

13. Medizinisches Operationssystem (50) nach Anspruch 12, **dadurch gekennzeichnet, dass** der Software-RCM-Roboterarm (20) zumindest sieben Freiheitsgrade aufweist.

14. Medizinisches Operationssystem (50) nach Anspruch 13, **dadurch gekennzeichnet, dass** sechs der Freiheitsgrade Rotationsfreiheitsgrade sind.

15. Medizinisches Operationssystem (50) nach einem der Ansprüche 12 bis 14, **gekennzeichnet durch:**
- einen zweiten Software-RCM-Roboterarm (18, 20), welcher entsprechend dem ersten Software-RCM-Roboterarm (14, 20) ausgebildet und mit der zweiten Roboterarmschnittstelle (16) gekoppelt sind.
